# FASCICULE DE BREVET EUROPEEN

(11) **EP 2 561 844 B1**
(45) Date de publication et mention de la délivrance du brevet: **20.01.2016**
(21) Numéro de dépôt: 12181197.0
(22) Date de dépôt: 21.08.2012
(51) Int. Cl.: A61F 13/02

(54) **Matériau composite comportant un support textile tricoté**
Composite material comprising a knitted textile support
Verbundmaterial, das eine textile Wirkwarenunterlage umfasst

(30) Priorité: 23.08.2011 US 201161526498 P
(43) Date de publication de la demande: 27.02.2013
(73) Titulaire: Zodiac Coating, 78370 Plaisir (FR)
(72) Inventeur: Guillo, Jean-Roger, 38300 SEREZIN DE LA TOUR (FR); Roland, Sophie, 38230 TIGNIEU (FR)
(74) Mandataire: Zimmermann, Alain

(56) Documents cités:
- WO-A1-87/05206
- US-A1- 2011 160 686

## Description

L'invention concerne un matériau composite comprenant une base textile et destiné à être intégré dans un dispositif médical, par exemple du type pansement. L'invention concerne également le procédé de fabrication dudit matériau composite ainsi qu'un dispositif médical le comprenant. Enfin, l'invention concerne l'utilisation d'un matériau à base textile dans la fabrication d'un dispositif médical, par exemple de type pansement, ou d'un produit intermédiaire de celui-ci.

On connaît des dispositifs médicaux qui sont appliqués sur une zone cutanée à protéger présentant une lésion, par exemple un pansement. Ces dispositifs permettent d'isoler la zone cutanée à protéger de toute contamination extérieure par des fluides environnants tels que l'eau, par des substances chimiques telles que le savon, les graisses, ainsi que par des agents de contamination bactérienne ou micro-organismes. Ces dispositifs permettent également d'évacuer vers l'extérieur l'excédent d'eau présent à intérieur dudit dispositif et issu des sécrétions de la zone cutanée à protéger. Enfin, ils permettent de protéger la zone cutanée recouverte de toute agression mécanique extérieure.

Généralement, un tel dispositif comprend une masse absorbante qui permet d'absorber les sécrétions de la zone cutanée à protéger, tels que les exsudats d'une plaie, une couche adhésive qui permet au dispositif d'adhérer à la zone cutanée péri-lésionnelle et d'être maintenu autour de cette zone, et éventuellement une couverture formant la surface extérieure dudit dispositif.

Parmi les adhésifs, les silicones présentent des propriétés particulièrement intéressantes : en effet ce sont des adhésifs « doux » qui ont pour avantage de permettre que le retrait du matériau composite ne soit pas douloureux tout en favorisant la cicatrisation.

Cependant, le silicone est difficile à mettre en oeuvre dès que l'on souhaite déposer une couche épaisse.

On connaît, par le document WO 87/05206, un pansement destiné être en contact avec une plaie, comprenant une structure ouverte, telle qu'un tricot, enduite d'un adhésif hydrophobe doux, tel qu'un silicone.

Ce pansement a pour avantage, grâce à la présence de l'adhésif doux, d'être bien toléré par la peau et de permettre le retrait du dispositif médical sans douleur pour le patient.

Toutefois, ce pansement a pour inconvénient d'être difficile à mettre en oeuvre en pratique. En effet, comme il ne peut absorber les exsudats de la plaie, il ne peut être employé seul et doit être utilisé conjointement avec une mousse absorbante et une bande de maintien, l'ensemble formant le dispositif médical.

Or, ces différents éléments ne peuvent être assemblés qu'au moment de la mise en place du dispositif, car ils ne présentent pas suffisamment d'adhésion entre eux pour permettre une cohésion dans la durée, ce qui nécessite également un remplacement fréquent du dispositif médical.

On connaît également le document OS 2011/0160686 qui décrit un dispositif médical perforé de manière uniforme ressemblant à un tamis qui n'offre qu'une seule fonctionnalité, permettre les échanges de fluide de façon homogène sur l'ensemble de la surface de la peau de la plaie du patient, cette fonctionnalité étant parfois insuffisante et inadaptée au type de plaie et de peau à soigner.

Le but de la présente invention est donc de fournir un matériau composite pour dispositif médical, destiné à venir en contact avec une plaie, qui puisse être utilisé avec une masse absorbante, facile à fabriquer et à intégrer dans un dispositif médical.

A cet effet, l'invention concerne un matériau composite comportant un support textile tricoté ouvert comportant un réseau de mailles, ledit support présentant au moins une zone vide de mailles ou dans laquelle la densité en maille est au moins 5 fois inférieure au reste du support, et un silicone imprégnant les mailles dudit support.

Les zones vides de mailles ou dans lesquelles la densité en maille est au moins 5 fois inférieure au reste du support (appelées par la suite zones de densité en maille peu élevée) pourront accueillir, lors de la mise en oeuvre d'un dispositif médical, une masse absorbante ou tout autre élément relativement volumineux.

Le matériau selon l'invention permet de bénéficier des avantages liés à l'emploi d'un adhésif doux qu'est le silicone tout en présentant une bonne cohésion.

Le matériau selon l'invention peut ainsi constituer un produit intermédiaire, facile à assembler avec d'autres produits intermédiaires pour obtenir un dispositif médical tel qu'un pansement.

Le support textile selon l'invention peut comporter des fils lui permettant d'avoir une capacité de déformation et une force de contention élevées. Par exemple, le textile tricoté comporte des filaments élastiques, tels que des fils comportant un polyuréthane de type élasthanne.

Selon un autre exemple, le support textile tricoté comporte des fils guipés ou texturés.

Le matériau composite selon l'invention peut comporter une ou plusieurs zones vides de mailles ou de densité en maille peu élevée, identiques ou différentes, pouvant présente une grande variété de forme. Par exemple, la au moins une zone vide de maille de densité en maille peu élevée, présente une forme choisie parmi les motifs suivants : circulaire, rectangulaire, carré ou ovoïde.

Le silicone peut imprégner une seule ou les deux faces du support du matériau.

Le silicone employé est de préférence un gel de silicone.

Selon un mode de réalisation préféré de l'invention, ledit silicone est un silicone adhésif. Ainsi, une fois assemblé au sein d'un dispositif médical, le matériau selon l'invention peut être directement appliqué sur la peau.

Selon un mode de réalisation de l'invention,ledit silicone s'étend continûment sur, la surface du support comprenant des mailles.

Alternativement, ledit silicone s'étend de façon discontinue sur la surface du support.

L'invention concerne également un dispositif médical pour protéger une zone cutanée, tel qu'un pansement, comportant le matériau décrit plus haut.

Typiquement, le dispositif médical peut comprendre d'autres produits intermédiaires tels qu'une membrane extérieure, une masse absorbante ou un tissu ajouré de type tulle faisant office d'interface avec la zone cutanée à protéger.

Les produits intermédiaires en contact avec le matériau composite de l'invention peuvent être assemblés avec ce dernier directement, si le silicone imprégnant le support est adhésif, ou au moyen d'une couche adhésive disposée entre eux.

Généralement, le dispositif médical selon l'invention comporte une membrane extérieure.

Cette membrane perméable à la vapeur d'eau mais imperméable aux fluides et aux micro-organismes constitue alors la couverture extérieure du dispositif médical.

Elle peut être respirante ou semi-respirante (perméable ou semi-perméable), et réalisée dans divers matériaux tels que le polyuréthane, un polyamide, le polyéthylène téréphtalate ou un silicone.

Les zones vides de mailles ou de densité en maille peu élevée du matériau composite de l'invention permettent de loger des éléments supplémentaires.

Le dispositif médical selon l'invention comporte une masse absorbante.

Cette masse absorbante est disposée dans une zone vide de maille ou dans laquelle la densité en maille est au moins 5 fois inférieure au reste du support du support textile.

On peut utiliser à cet effet des masses absorbantes bien connues dans le domaine, par exemple une mousse hydrophile à base de polyuréthane ou des fibres hydrocolloïdes.

Eventuellement, notamment lorsque le dispositif médical est destiné à protéger une zone cutanée présentant une plaie, la masse absorbante peut être imprégnée par un principe actif permettant de traiter ladite plaie.

L'invention porte également sur un procédé de fabrication du matériau composite décrit plus haut.

Ainsi, le procédé selon l'invention comporte les étapes consistant à :
- tricotersur un dispositif à levée d'aiguilles de type Jacquard un support textile présentant des zones vides de mailles ou dans lesquelles la densité en maille est au moins 5 fois inférieure au reste du support,
- déposer sur au moins une face de dudit support textile une solution de pré-polymère de silicone de façon à imprégner ledit support, et
- polymériser ladite solution de pré-polymère.

Le procédé selon l'invention est de mise en oeuvre aisée.

En effet, les techniques de tricotage Jacquard sont connues et bien maîtrisées et permettent d'obtenir facilement un tricot pouvant présenter une grande variété de motifs, par exemple des ajours (zones vides de mailles) ou des zones de faible densité en maille de taille et de forme très variées.

L'étape de dépôt de la solution de pré-polymère peut être réalisée selon différents procédés bien connus de l'homme du métier, en particulier par enduction à la racle, au cylindre ou par foulardage.

Selon les applications envisagées, on pourra enduire l'une ou les deux faces du tricot avec la solution de pré-polymère de silicone.

De préférence, ledit procédé comprend en outre une étape consistant à désobstruer les zones vides de mailles ou dans lesquelles la densité en maille est au moins 5 fois inférieure au reste du dudit support textile remplies de la solution de pré-polymère.

En effet, selon la viscosité de la solution de pré-polymère employée, les zones vides de mailles peuvent être obstruées, ce qui rendrait difficile l'intégration future d'une masse absorbante, par exemple.

L'opération de désobstruction peut être réalisée selon différentes techniques, en particulier, égouttage de la solution de prepolymeres ou bien soufflage à l'aide d'une lame d'air sous pression, soufflant transversalement au sens de défilement de la nappe textile, avec différents angles d'incidence.

L'étape de polymérisation est de préférence réalisée en introduisant le tricot imprégné de la solution de pré-polymère dans un four à air chaud.

On obtient alors une couche de silicone polymérisé à la surface du support tricoté, cette couche étant continue sauf dans les zones vides de mailles ou présentant une densité en maille au moins 5 fois inférieure au reste du support textile.

De préférence, la quantité de silicone polymérisé est de 50 à 300 g/m² sur la ou les faces enduites.

Une fois le matériau fabriqué, on peut le recouvrir d'un film ou d'un papier anti-adhérent destiné à la protéger de toute agression, puis l'enrouler en bobine.

Il est alors facilement conservé puis déplacé en vue de son intégration lors de la fabrication d'un dispositif médical tel qu'un pansement.

Enfin, l'invention porte sur l'utilisation d'un produit intermédiaire du matériau composite décrit plus haut, dans la fabrication de dispositifs médicaux.

A cet effet, l'invention concerne l'utilisation d'un matériau comportant un support textile tricoté ouvert présentant au moins une zone vide de mailles et/ou dans laquelle la densité en maille est au moins 5 fois inférieure au reste de la couche support, pour la fabrication d'un dispositif médical pour protéger une zone cutanée ou pour la fabrication d'un produit intermédiaire de celui-ci.

L'invention va maintenant être décrite plus en détails à l'aide des dessins annexés dans lesquels :
- la figure 1 représente une vue de dessus d'un matériau selon un premier mode de réalisation,
- la figure 2 représente une vue de dessus d'un matériau selon un autre mode de réalisation,
- la figure 3 représente une vue en coupe d'un dispositif médical, et
- la figure 4 représente une vue en coupe d'un dispositif médical selon l'invention.

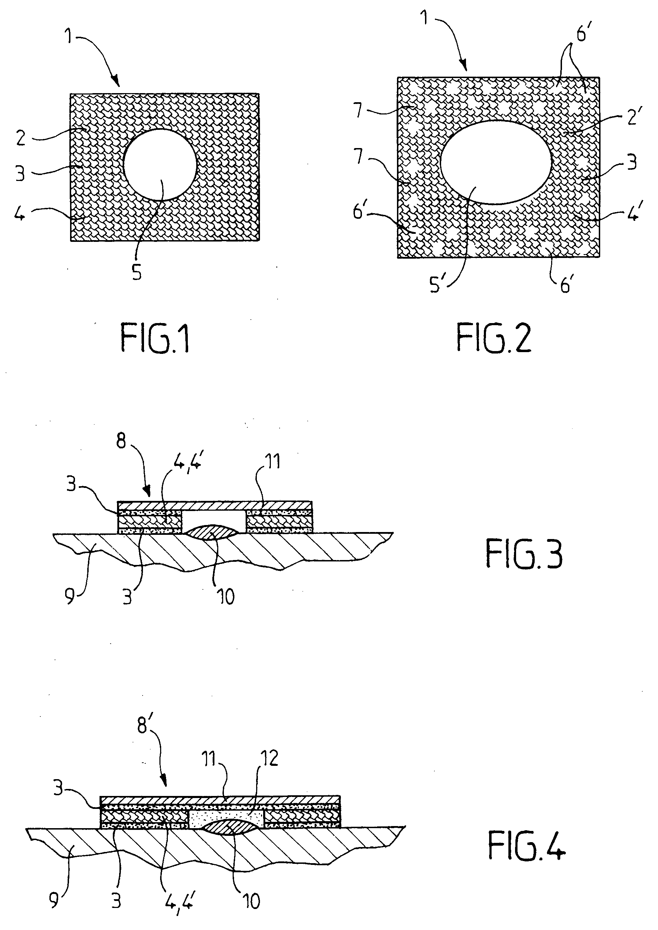

La figure 1 représente un matériau composite 1, destiné à être incorporé dans un dispositif médical, comprenant une couche support 2 et un gel de silicone 3.

La couche support est formée d'un réseau de mailles 4 tricotées à partir d'un fil élastique, permettant au matériau de présenter de grandes capacités de déformation.

Au sein de cette couche support se trouve une zone vide de mailles 5 circulaire.

La forme de cette zone vide de maille 5, est adaptée pour pouvoir éventuellement recevoir un élément du dispositif médical dans lequel le matériau 1 est destiné à être intégré, tel qu'une masse absorbante.

Les mailles 4 de ladite couche support 2 sont imprégnées d'un gel de silicone adhésif 3, de façon à ce que les deux faces de la couche support 2 soient imprégnées, la zone vide de mailles étant dépourvue de gel de silicone 3.

Le matériau composite 1 ainsi obtenu pourra ensuite être assemblé avec un ou plusieurs produits intermédiaires pour former un dispositif médical, dont ledit matériau constituera l'interface avec la zone cutanée à protéger sur laquelle le dispositif sera disposé.

La figure 2 représente une alternative du matériau présenté dans la figure 1, dans laquelle la couche support 2' formée d'un réseau de mailles 4' comporte non seulement une zone vide de mailles 5' circulaire, mais également plusieurs zones 6', dans lesquelles la densité en mailles 4' est au moins 5 fois inférieure au reste de la couche support 2', de sorte que ces zones 6', de par leur faible densité en mailles forment de petites ouvertures 7 entre les mailles 4'.

La figure 3 représente un dispositif médical 8 selon l'invention, comportant le matériau composite 1 présenté dans la figue 1.

Le dispositif médical 8, ici un pansement destiné à être appliqué sur une zone cutanée à protéger 9 présentant une plaie 10, comporte le matériau composite 1 et une membrane semi-perméable 11 assemblés ensemble.

Le dispositif 8 est disposé de façon à ce que le matériau composite 1 vienne directement en contact avec la zone cutanée à protéger tandis quela zone vide de maille 5 est située en regard de la plaie 10, le gel de silicone adhésif 3 permettant une adhésion atraumatique avec ladite zone cutanée 9.

Le matériau composite 1 est assemblé avec une membrane semi-perméable 11, par exemple à base de silicone, constituant lacouverture extérieure du dispositif 8.

Le matériau composite 1 et la membrane semi-perméable 11 sont maintenus ensemble grâce au gel de silicone adhésif 3 imprégnant les deux faces du matériau 1.

Ainsi le pansement 8, permet tout en protégeant la zone cutanée 9 ainsi que la plaie 10, d'évacuer les exsudats libérés par cette dernière via la membrane semi-perméable 11.

La figure 4 présente une alternative du dispositif médical présenté à la figure 3, dans lequel le dispositif 8' comprend le matériau composite 1 et une membrane semi-perméable, maiségalement une masse absorbante 12, disposée au sein de la zone vide de mailles 5.

Ainsi, la masse absorbante vient directement au contactde la plaie 10, et absorbe les exsudats générés par cette dernière.

Cette variante est particulièrement adaptée lorsque la zone cutanée à protéger présente une plaie exsudative, et permet d'éviter que les exsudats ne s'accumulent à la surface de la plaie, nuisant à sa cicatrisation.

## Revendications

1. Dispositif médical pour protéger une zone cutanée, tel qu'un pansement, comprenant :
- un matériau composite (1, 8, 8') comportant un support textile tricoté (2, 2') ouvert muni d'un réseau de mailles (4, 4'), ledit support textile (2, 2') présentant au moins une zone vide de mailles (5, 5' et/ou 6') ou dans laquelle la densité en maille est au moins 5 fois inférieure au reste du support, et un silicone (3) imprégnant ledit support ; et
- une masse absorbante (12) disposée dans la zone vide de maille (5, 5' et/ou 6') ou dans laquelle la densité en maille est au moins 5 fois inférieure au reste du support du support textile.

2. Dispositif médical selon la revendication 1, **caractérisé en ce qu'**il comporte en outre une membrane extérieure (11).

3. Dispositif médical selon l'une quelconque des revendications précédentes **caractérisé en ce que** le support textile tricoté (2, 2') comporte des filaments élastiques

4. Dispositif médical selon l'une quelconque des revendications précédentes **caractérisé en ce que** le support textile tricoté (2, 2') comporte des fils guipés ou texturés.

5. Dispositif médical selon l'une quelconque des revendications précédentes, **caractérisé en ce que** la au moins une zone vide de maille ou dans laquelle la densité en maille est au moins 5 fois inférieure au reste du support, présente une forme choisie parmi les motifs suivants : circulaire, rectangulaire, carré ou ovoïde.

6. Dispositif médical selon l'une quelconque des revendications précédentes, **caractérisé en ce que** ledit silicone est un silicone adhésif.

7. Dispositif médical selon l'une quelconque des revendications précédentes, **caractérisé en ce que** ledit silicone s'étend continûment sur la surface du support.

8. Dispositif médical selon l'une quelconque des revendications 1 à 6, **caractérisé en ce que** ledit silicone s'étend de façon discontinue sur la surface du support.

9. Procédé de fabrication d'un dispositif médical selon l'une quelconque des revendications 1 à 8, comportant les étapes consistant à :
- tricoter sur un dispositif à levée d'aiguilles de type Jacquard un support textile présentant des zones vides de mailles ou dans lesquelles la densité en maille est au moins 5 fois inférieure au reste du support,
- déposer sur au moins une face de dudit support textile une solution de pré-polymère de silicone de façon à imprégner ledit support,
- polymériser ladite solution de pré-polymère, et
- disposer une masse absorbante (12) dans la zone vide de maille (5, 5' et/ou 6') ou dans laquelle la densité en maille est au moins 5 fois inférieure au reste du support du support textile.

10. Procédé de fabrication selon la revendication 9, **caractérisé en ce que** l'étape de dépôt de la solution de pré-polymère est réalisée par enduction à la racle, au cylindre ou par foulardage.

11. Procédé selon la revendication 9 ou la revendication 10, **caractérisé en ce que** ledit procédé comprend en outre une étape consistant à désobstruer les ouvertures dudit support textile remplies de la solution de pré-polymère.

## Patentansprüche

1. Medizinische Vorrichtung zum Schutz eines Hautbereichs wie ein Pflaster, umfassend:
- ein Verbundmaterial (1, 8, 8'), das eine offene Wirkwarenunterlage (2, 2'), die mit einem Maschennetz (4, 4') versehen ist, wobei die Wirkwarenunterlage (2, 2') mindestens einen Bereich (5, 5' und/oder 6') aufweist, in dem keine Maschen vorliegen oder in dem die Maschendichte im Vergleich zu der restlichen Unterlage mindestens 5 Mal geringer ist, und ein die Unterlage tränkendes Silikon (3) umfasst, und
- ein saugfähiges Material (12), das in dem Bereich (5, 5' und/oder 6') angeordnet ist, in dem keine Maschen vorliegen oder in dem die Maschendichte im Vergleich zu der restlichen Unterlage der Wirkwarenunterlage mindestens 5 Mal geringer ist.

2. Medizinische Vorrichtung nach Anspruch 1, **dadurch gekennzeichnet, dass** sie ferner eine äußere Membran (11) umfasst.

3. Medizinische Vorrichtung nach einem der vorhergehenden Ansprüche, **dadurch gekennzeichnet, dass** die Wirkwarenunterlage (2, 2') elastische Filamente umfasst.

4. Medizinische Vorrichtung nach einem der vorhergehenden Ansprüche, **dadurch gekennzeichnet, dass** die Wirkwarenunterlage (2, 2') Coregarne oder texturierte Garne umfasst.

5. Medizinische Vorrichtung nach einem der vorhergehenden Ansprüche, **dadurch gekennzeichnet, dass** der mindestens eine Bereich, in dem keine Maschen vorliegen oder in dem die Maschendichte im Vergleich zu der restlichen Unterlage mindestens 5 Mal geringer ist, eine Form aufweist, die aus folgenden Mustern ausgewählt ist: kreisförmig, rechteckig, quadratisch oder eiförmig.

6. Medizinische Vorrichtung nach einem der vorhergehenden Ansprüche, **dadurch gekennzeichnet, dass** das Silikon ein Klebesilikon ist.

7. Medizinische Vorrichtung nach einem der vorhergehenden Ansprüche, **dadurch gekennzeichnet, dass** sich das Silikon durchgehend über die Oberfläche der Unterlage erstreckt.

8. Medizinische Vorrichtung nach einem der Ansprüche 1 bis 6, **dadurch gekennzeichnet, dass** sich das Silikon nicht durchgehend über die Oberfläche der Unterlage erstreckt.

9. Verfahren zur Herstellung einer medizinischen Vorrichtung nach einem der Ansprüche 1 bis 8, umfassend die folgenden Etappen:
- Stricken einer Wirkwarenunterlage, die Bereiche aufweist, in denen keine Maschen vorliegen oder in denen die Maschendichte im Vergleich zu der restlichen Unterlage mindestens 5 Mal geringer ist, auf einer Jacquard-Nadelhubvorrichtung,
- Auftragen einer Silikonpräpolymerlösung auf mindestens eine Fläche der Wirkwarenunterlage, um die Unterlage zu tränken,
- Polymerisieren der Präpolymerlösung und
- Auftragen eines saugfähigen Materials (12) in dem Bereich (5, 5' und/oder 6'), in dem keine Maschen vorliegen oder in dem die Maschendichte im Vergleich zu der restlichen Unterlage der Wirkwarenunterlage mindestens 5 Mal geringer ist.

10. Herstellungsverfahren nach Anspruch 9, **dadurch gekennzeichnet, dass** der Schritt des Auftragens der Präpolymerlösung durch Rakeln, Walzenauftrag oder Foulardierung erfolgt.

11. Verfahren nach Anspruch 9 oder Anspruch 10, **dadurch gekennzeichnet, dass** das Verfahren ferner einen Schritt umfasst, der aus dem Freilegen der mit der Präpolymerlösung gefüllten Öffnungen der Wirkwarenunterlage besteht.

## Claims

1. Medical device for protecting a cutaneous zone, such as a dressing, comprising:
- a composite material (1, 8, 8') comprising an open knitted textile support (2, 2') provided with a network of stitches (4, 4'), the said textile support (2, 2') exhibiting at least one zone devoid of stitches (5, 5' and/or 6') or in which the density of stitches is at least 5 times lower than the rest of the support, and a silicone (3) impregnating the said support, and
- an absorbent mass (12) disposed in the zone devoid of stitches (5, 5' and/or 6') or in which the density of stitches is at least 5 times lower than the rest of the textile support.

2. Medical device according to claim 1, **characterised in that** it also comprises an external membrane (11).

3. Medical device according to any one of the preceding claims, **characterised in that** the knitted textile support (2, 2') comprises elastic filaments.

4. Medical device according to any one of the preceding claims, **characterised in that** the knitted textile support (2, 2') comprises lapped or textured threads.

5. Medical device according to any one of the preceding claims, **characterised in that** the at least one zone devoid of stitches or in which the density of stitches is at least 5 times lower than the rest of the support exhibits a shape selected from the following patterns: circular, rectangular, square or ovoid.

6. Medical device according to any one of the preceding claims, **characterised in that** the said silicone is an adhesive silicone.

7. Medical device according to any one of the preceding claims, **characterised in that** the said silicone extends continuously over the surface of the support.

8. Medical device according to any one of claims 1 to 6, **characterised in that** the said silicone extends discontinuously over the surface of the support.

9. Method of production of a medical device according to any one of claims 1 to 8, comprising the steps consisting of:
- knitting on a needle-lifting device of Jacquard type a textile support exhibiting zones devoid of stitches or in which the density of stitches is at least 5 times lower than the rest of the support,
- depositing on at least one face of the said textile support a silicone pre-polymer solution in such a way as to impregnate the said support,
- polymerising the said pre-polymer solution, and
- disposing an absorbent mass (12) in the zone devoid of stitches (5, 5' and/or 6') or in which the density of stitches is at least 5 time lower than the rest of the textile support.

10. Method of production according to claim 9, **characterised in that** the step of depositing the pre-polymer solution is carried out by coating with a doctor, cylinder or by sizing.

11. Method according to claim 9 or claim 10, **characterised in that** the said method also comprises a step consisting of unblocking the openings of the said textile support filled with the pre-polymer solution.
